(19) Europäisches Patentamt
European Patent Office
Office européen des brevets

(11) Publication number : **0 504 109 A1**

# (12) EUROPEAN PATENT APPLICATION

(21) Application number : **92810163.3**

(51) Int. Cl.$^5$ : **C07D 405/14,** C08G 73/10

(22) Date of filing : **03.03.92**

(30) Priority : **11.03.91 US 667728**

(43) Date of publication of application :
**16.09.92 Bulletin 92/38**

(84) Designated Contracting States :
**BE DE ES FR GB IT NL SE**

(71) Applicant : **CIBA-GEIGY AG**
**Klybeckstrasse 141**
**CH-4002 Basel (CH)**

(72) Inventor : **Blyakhman, Yefim**
**4705 Henry Hudson Pkway**
**Bronx, NY 10471 (US)**

## (54) Aromatic monoanhydride-esters.

(57) Aromatic monoanhydride-esters of the formula (I)

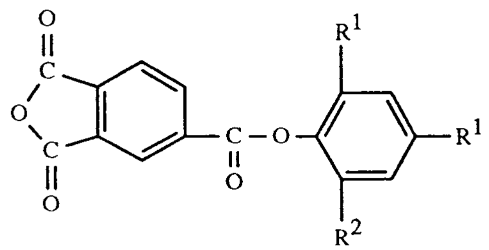

(I) ,

wherein $R^1$ is

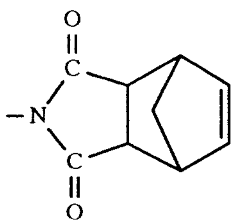

,

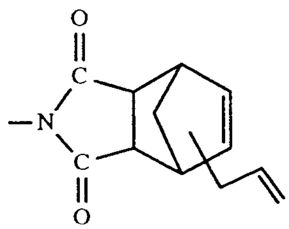

or

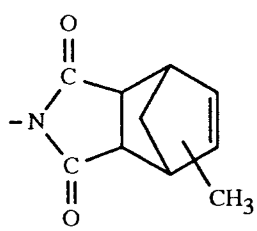

and $R^2$ is hydrogen, $C_1$-$C_4$alkyl or $-OCH_3$ are useful as endcapping agents for the synthesis of multifunctional high temperature resins such as polyimides, polyimide-esters, polyimide-ethers and polyimide-amides.

EP 0 504 109 A1

Addition type thermoset polyimide, polyimide-amide, polyimide-ester resins are finding use as matrix resins for advanced composites, adhesives, coating and other applications. These resins generally contain two reactive end groups per molecule such as acetylene, norbonene and maleimide. In order to increase the glass transition temperature and improve thermal performance of cured resin, it is desirable to increase functionality of the resins.

One approach is the use of endcapping agents having two unsaturated groups in the molecule as taught in U.S. Patent No. 4,233,220 wherein bis-maleimidophthalic anhydride of the formula

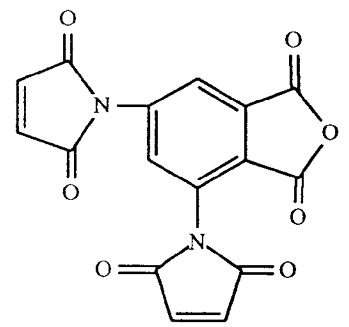

is disclosed. This compound could be used for the synthesis of tetrafunctional polyimides with bis-maleimido phenyl end groups but the synthesis of the monomer is a complex multistep process resulting in a low yield of the product (<20%). Additionally, attachment of anhydride and both maleimido groups to the same nucleus creates steric hindrance and reduces reactivity of the end groups in the curing process.

Accordingly, it is an object of the present invention to provide new multifunctional monomers which can be used for the synthesis of reactive polyimides, polyimide-esters, polyimide-ethers and the like having increased functionality and, thus, improved glass transition temperature.Various other objects and advantages of this invention will become apparent from the following description thereof.

The present invention relates to aromatic mono-anhydride-esters having two unsaturated reactive groups in a molecule for use as endcapping agents in the synthesis of multifunctional high temperature resins.

The present invention relates to aromatic monoanhydride-esters of the formula I

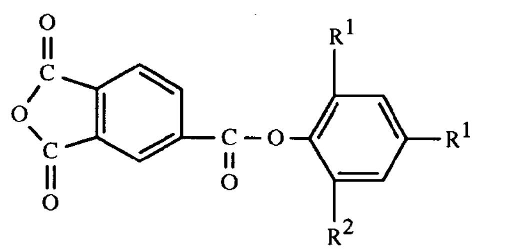

(I),

wherein $R^1$ is

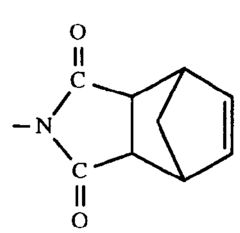

,

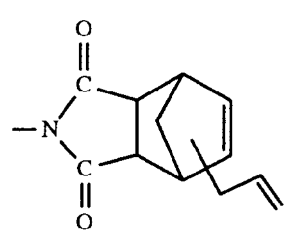

or

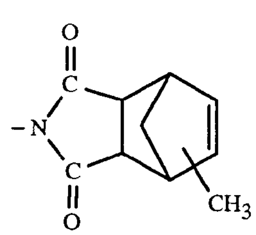

and $R^2$ is hydrogen, $C_1$-$C_4$alkyl or $-OCH_3$; preferably $R^2$ is hydrogen.

The monoanhydride-esters of the present invention can be prepared by reaction of trimellitic anhydride chloride with phenols having two unsaturated groups as exemplified in the reaction scheme below:

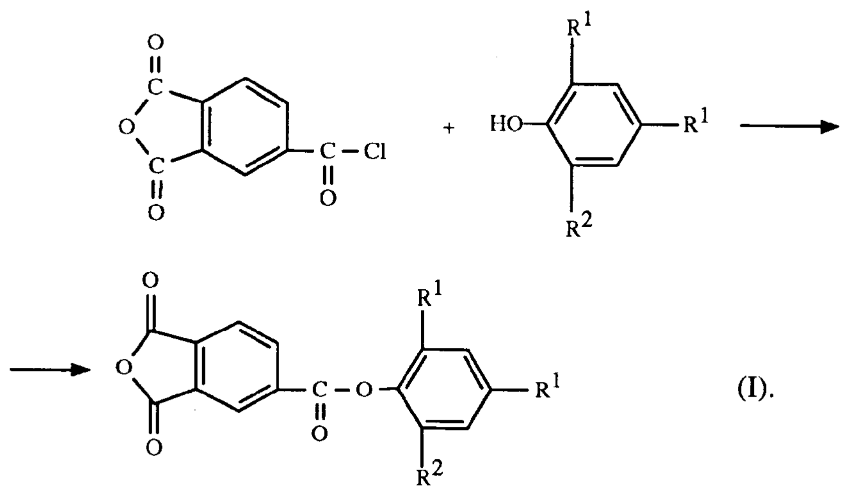

(I).

Phenols having two unsaturated imido (nadic, methyl nadic, allylnadic) groups in a molecule may be obtained by reaction of diaminophenol with corresponding anhydride.

Reaction between trimellitic anhydride chloride and the phenols may be carried out in anhydrous solvents, such as ether, tetrahydrofuran, acetone, methylethyl ketone, benzene, toluene, ethylene dichloride etc., in the presence of tertiary amines such for instance pyridine or triethylamine.Preferably, the tertiary amine is added to a solution of trimellitic anhydride chloride and a phenol cooled to 0-5°C and subsequent stirring of the reaction mixture at 15-75°C for 0.5-6 hrs. The obtained anhydride-ester may be separated by precipitation in a solvent in which tertiary amines' salts are soluble, e.g., methanol, ethanol, 2-propanol.

The multifunctional high temperature resins contain, per molecule, on average 2-3 aromatic monoanhydride-ester groups of the formula I set forth hereinabove.

The polymers according to the present invention can be manufactured according to methods of synthesis which are known for the manufacture of macromolecules which have pendant side groups. In principle, the polyimides, polyimide-esters, polyimide-ethers, polyimide-amides and routes disclosed in U.S. Patent No. 4,079,041, which is hereby incorporated by reference, can be used.

The following examples serve to give specific illustration of the practice of this invention, but they are not intended in any way to limit the scope of this invention.

Example 1: Synthesis of 2,4-dinadicimidophenol

315.2 g (1.6 mol) of 2,4-diaminophenol dihydrochloride are dissolved in 2 lt of N,N-dimethylacetamide (DMAc) and neutralized by 268.8 g (3.2 mol) of $NaHCO_3$ at 60°C under $N_2$. After that the mixture is cooled to room temperature and 525.2 g (3.2 mol) of 5-norbornene-2,3-dicarboxylic (nadic) anhydride and 400 ml of toluene are added. The reaction mixture is refluxed with a Dean-Stark trap to remove water. Then toluene is distilled off, the reaction mixture is filtered, and the filtrate precipitated in water at 1:5 ratio. The precipitate is washed with 3 times and dried in a vacuum-oven at 90°C. 2,4-dinadicimido phenol has a melting point of 263-265°C, its IR and NMR spectra are consistent with proposed structure and its purity is 95% by HPLC.

Example 2: Synthesis of Monoanhydride-ester of the Following Structure

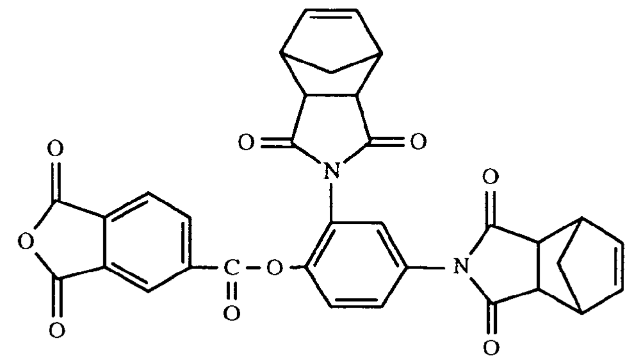
(Ia).

To a solution of 105.3 g (0.5 mol) of trimellitic anhydride chloride and 208.4 g (0.5 mol) of 2,4-dinadicimido phenol in 2 lt of dry acetone cooled to 0°C 43.5 g (0.55 mol) of pyridine are added dropwise (30 min). After that the reaction mixture is stirred 2 hrs. at 20°C and 1 hr. at 50-55°C. The mixture is cooled to 15°C and filtered. The filtrate is mixed with 4 lt of anhydrous isopropanol in a blender and formed precipitate is washed with ether and dried in a vacuum over at 80°C. Yield is 280 g (95%). Obtained product -a white powder - has a melting point of 276-277°C. Its IR and NMR spectra are consistent with proposed structure. N-phenyl imide of the anhydride is obtained by reaction of 1 mole of the anhydride with 1 mole of aniline. Structure of the derivative is confirmed by IR and NMR spectra.

Example 3: Synthesis of polyimide-ester Monomers used:

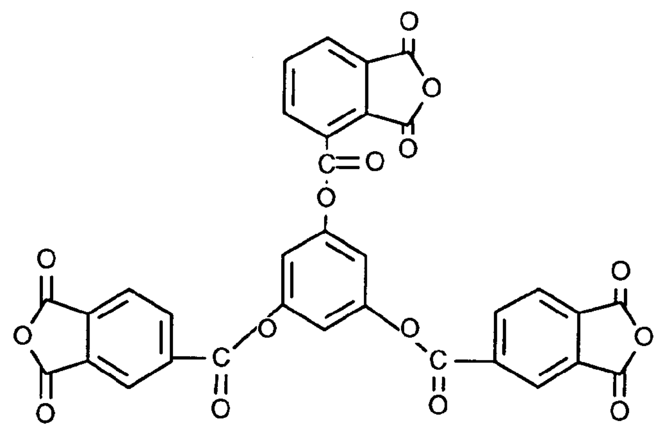
Trisanhydride (II)

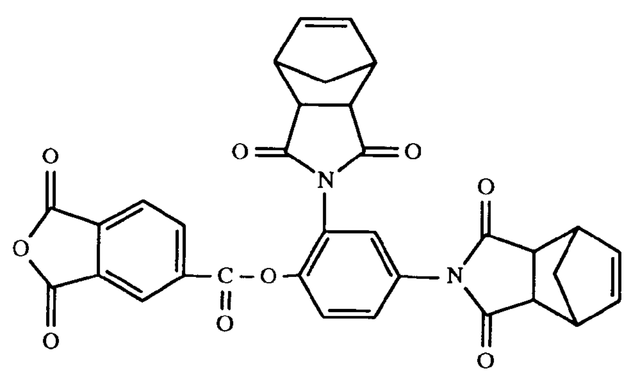
Monoanhydride-ester having two nadic imido groups (Ia)

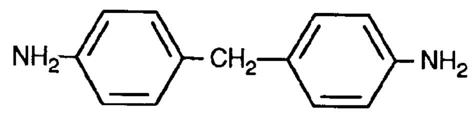

Methylenedianiline (III)

A solution of 59.5g (0.3 mol) of methylenedianaline (III) in 1200 ml of DMAc is cooled to 0°C and 64.8g (0.1 mol) of the trisanhydride II is added over 1.5 hrs. in 10g portions under $N_2$ with vigorous stirring while the temperature is maintained below 0°C. 177.8 g (0.3 mol) of the monoanhydride Ia is then added and the reaction mixture stirred overnight at room temperature. To the resulting solution, 500 ml of toluene is added and the mixture refluxed with a Dean-Stark trap for 3 hrs. Then, the toluene is distilled off and the solution heated at 163-165° for 5 hrs. After cooling, the solution is precipitated in 5 lt of methanol and the obtained yellow powder is dried in a vacuum-oven. The yield of the polyimide-ester is 178g. The polymer exhibits a $T_g$ of 223°C (determined by DSC) and an intrinsic viscosity of 0.08 dl/g (NMP, 25°C). The polyimide-ester undergoes polymerization upon heating to 300°C.

Example 4: Synthesis of 2,4-di(methylnadicimido)phenol

197.1 g (1 mole) of 2,4-diaminophenol dihydrochloride is dissolved in 2 liters of DMAc and neutralized with 168 g (2 moles) of $NaHCO_3$ at 60°C under $N_2$. The mixture is then cooled to room temperature and 356.4 g (2 moles) of methyl-5-norbomene-2,3-dicarboxylic anhydride and 300 ml of toluene are added. The reaction mixture is refluxed with a Dean-Stark trap to remove water. After that toluene is distilled off, the reaction mixture is filtered and the filtrate precipitated in water at a 1:7 ratio. The precipitate is washed 5 times with water and dried in a vacuum-oven at 80°C. The yield is 404.8 g (91%) and the structure of the obtained 2,4-di(methylnadicimido)phenol is confirmed by IR and NMR and its purity is 93% by HPLC.

Example 5: Synthesis of Monoanhydride-ester of the Following Structure

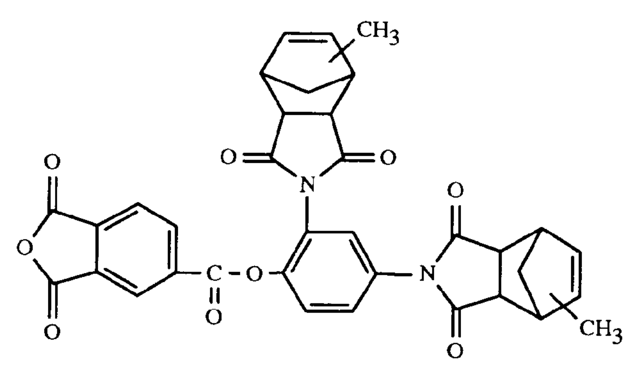

To a solution of 210.6 g (1 mol) of trimellitic anhydride chloride and 444.8 g (1 mol) of 2,4-di(methylnadicimido)phenol in 3.5 liters of dry acetone cooled to 0°, 111.3 g (1.1 mol) of triethylamine are added dropwise (1 hr). After that the reaction mixture is stirred 2.5 hrs at room temperature and 1 hr at 50-55°C. The mixture is cooled to 10°C and filtered. The filtrate is mixed with 6 liters of anhydrous isopropanol and the precipitate formed is washed with ether and dried in a vacuum-oven at 60°C. The yield is 569.4 g (92%). The IR and NMR spectra of the obtained product are consistent with the proposed structure. N-phenylimide of the monoanhydride-ester is synthesized by reaction of 1 mole of the anhydride with 1 mole of aniline. The structure of the derivative is confirmed by IR and NMR.

## Claims

**1.** An aromatic monoanhydride-ester of the formula I

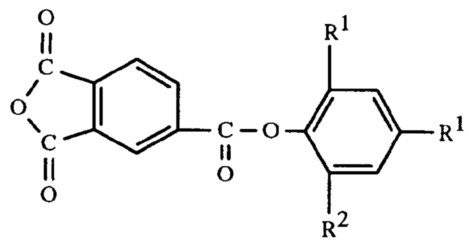 (I),

wherein $R^1$ is

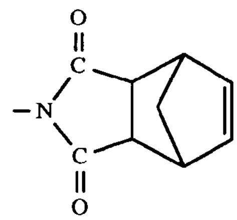 ,

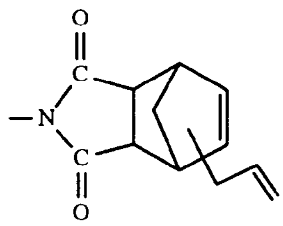 or

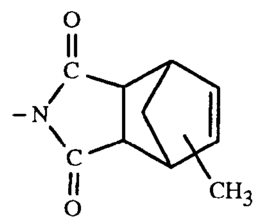

and $R^2$ is hydrogen, $C_1$-$C_4$alkyl or $-OCH_3$.

**2.** An aromatic monoanhydride-ester according to claim 1 wherein $R^2$ is hydrogen.

**3.** A multifunctional high temperature resin comprising a polymer selected from the group consisting of polyimides, polyimide-esters, polyimide-ethers and polyimide-amides comprising radicals of an aromatic monoanhydride-ester according to claim 1 as endcapping agents.

**4.** A multifunctional high temperature resin according to claim 3 wherein said polymer is a polyimide.

**5.** A multifunctional high temperature resin according to claim 3 wherein said polymer is a polyimide-ester.

**6.** A multifunctional high temperature resin according to claim 3 wherein said polymer is a polyimide-ether.

**7.** A multifunctional high temperature resin according to claim 3 wherein said polymer is a polyimide-amide.

**8.** A multifunctional high temperature resin comprising a polymer selected from the group consisting of polyimides, polyimide-esters, polyimide-ethers and polyimide-amides comprising radicals of an aromatic monoanhydride-ester according to claim 2 as endcapping agents.

European Patent Office

# EUROPEAN SEARCH REPORT

Application Number

EP 92 81 0163

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | US-A-4 233 220 (CIBA-GEIGY CORPORATION)<br>* complete document *<br>--- | 1,8 | C07D405/14<br>C08G73/10 |
| Y | EP-A-0 323 540 (THE BOEING COMPANY)<br>* PAGE 1-6 *<br>--- | 1,8 | |
| Y | EP-A-0 289 798 (THE BOEING COMPANY)<br>* PAGE 1-4 *<br>--- | 1,8 | |
| A | EP-A-0 277 476 (THE BOEING COMPANY)<br>* PAGE 1 - 7 *<br>--- | 1,8 | |
| A | EP-A-0 152 372 (CIBA-GEIGY AG)<br>* PAGE 1-3 *<br>--- | 1,8 | |
| A | EP-A-0 355 435 (GENERAL ELECTRIC COMPANY)<br>* PAGE 1-5 *<br>----- | 1,8 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 04 JUNE 1992 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)